# EUROPEAN PATENT APPLICATION

(11) **EP 4 223 124 A1**
(43) Date of publication of application: **09.08.2023**
(21) Application number: 23153764.8
(22) Date of filing: 27.01.2023
(51) Int. Cl.: A01N 41/04, A01P 1/00

(54) **ANTIMICROBIAL WIPE**

(30) Priority: 02.02.2022 US 202263267454 P
(71) Applicant: Kraton Polymers Nederland B.V., 1322 CE Almere (NL)
(72) Inventor: KRUTZER, Bert, Houston, Texas 77084 (US); WRIGHT, Kathryn J, Houston, Texas 77084 (US); SMITH, James, Houston, Texas 77084 (US); ALWATTARI, Ali, Houston, Texas 77084 (US)
(74) Representative: Henkel & Partner mbB

(57) **Abstract**

An antimicrobial wipe is disclosed comprising a substrate and an antimicrobial composition. The antimicrobial composition contains a sulfonated block copolymer having an ion exchange capacity (IEC) of > 0.5 meq/g, and (ii) a solvent system. The solvent system contains a mixture of two or more solvents selected from the group consisting of organic solvents, inorganic solvents, terpene-based compounds, and decarboxylated rosin acids. The antimicrobial wipe is in the form of a fabric, a sponge, a toilette, and the like. The sulfonated block copolymer is dissolved in the solvent system, such that when the antimicrobial wipe is in contact with a target surface, the sulfonated block copolymer is imparted from the antimicrobial wipe onto the surface forming a coating layer. The coating layer causes a reduction in a microbe concentration by at least 1 logic CFU (colony forming units) within 120 minutes upon contact.

## Description

### TECHNICAL FIELD

The disclosure relates to antimicrobial wipes for protecting surfaces prone to microbial contamination, and methods of preparation thereof.

### BACKGROUND

Microbes share the environment of human. A microbe is generally defined to be an organism from which growth and development to be expected, including viruses, bacteria, mold, fungus, algae, etc., some with the capacity to cause sickness and infection.

A variety of chemicals have been used as antimicrobial agents or cleaning agents, e.g., antimicrobial cleaning solutions with alcohol and quaternary ammonium compounds, or disinfectant-impregnated wipes (DIWs) containing any of alcohol and quaternary ammonium compounds, hydrogen peroxide, hypochlorite, chlorine dioxide, etc., to effectively kill microbes. Some disinfectant wipes and spray cleans have instructions for the cleaning agents to stay on a surface from 30 sec. to 4 min., or even as long as 10 min., to effectively kill germs. The efficacy of cleaning solutions and DIWs is short-term, i.e., as a point-in-time disinfectant with little if any residual effect. Additionally, DIWs are typically discarded after just one use.

A need exists for an antimicrobial wipe which can provide long-term residual protection for surfaces from microbes, and sustainable - can be used more than once. A need also exists for an antimicrobial wipe that provides instant antimicrobial (upon contact) as well as residual or long-term efficacy.

### SUMMARY

In a first aspect, the disclosure relates to an antimicrobial wipe for protecting a surface, comprising, consisting essentially of, or consisting of a wet wipe having a substrate impregnated with a sufficient amount of an antimicrobial composition. The antimicrobial composition comprises (i) 0.1 to 50 wt.% of a sulfonated block copolymer having an ion exchange capacity (IEC) of at least 0.5 meq/g, and (ii) 50 to 99.90 wt.% of a solvent system. The solvent system contains a mixture of two or more solvents selected from the group consisting of organic solvents, inorganic solvents, terpene-based compounds, and decarboxylated rosin acids. Each solvent has at least one of: a) at least one OH group, b) at least one ester and / or ether group, and c) 10 or more carbon atoms. The antimicrobial wipe after wiping with a shear force onto the surface to be protected, forms a coating layer. The coating layer causes reduction in a microbe concentration by at least 1 log₁₀ CFU (colony forming units) within 120 minutes upon contact with the coating layer.

In a second aspect, an antimicrobial wipe for protecting a surface comprising a dry wipe having a substrate impregnated with a sufficient amount of a sulfonated block copolymer having an ion exchange capacity (IEC) of at least 0.5 meq/g. The substrate containing the sulfonated block copolymer after being in contact with a solvent system pre-applied onto the surface to be protected or the substrate itself, is dissolved into a solvent system for the sulfonated block copolymer to be imparted from the substrate onto the surface upon contact, forming a coating layer. The solvent system contains a mixture of two or more solvents selected from the group consisting of organic solvents, inorganic solvents, terpene-based compounds, and decarboxylated rosin acids. Each solvent has at least one of: a) at least one OH group, b) at least one ester and / or ether group, and c) 10 or more carbon atoms. The coating layer causes reduction in a microbe concentration by at least 1 logic CFU (colony forming units) within 120 minutes upon contact with the coating layer.

In a third aspect, a method for protecting a surface, comprising providing an wet wipe having a substrate impregnated with a sufficient amount of an antimicrobial composition comprising a sulfonated block copolymer having an ion exchange capacity (IEC) of at least 0.5 meq/g, and a solvent system. The sulfonated block copolymer is dissolved in the solvent system containing a mixture of two or more solvents selected from the group consisting of organic solvents, inorganic solvents, terpene-based compounds, and decarboxylated rosin acids. Each solvent has at least one of: a) at least one OH group, b) at least one ester and / or ether group, and c) 10 or more carbon atoms. Wiping the surface with the wet wipe by shear force for the antimicrobial composition to be imparted from the wet wipe onto the surface, forming a coating layer. The coating layer causes reduction in a microbe concentration by at least 1 log10 CFU (colony forming units) within 120 minutes upon contact with the coating layer.

In a fourth aspect, a method for protecting a surface, comprising: providing a dry wipe having a substrate impregnated with a sufficient amount of a sulfonated block copolymer having an ion exchange capacity (IEC) of at least 0.5 meq/g. Pre-applying a solvent system onto the surface to be protected. The solvent system contains a mixture of two or more solvents selected from the group consisting of organic solvents, inorganic solvents, terpene-based compounds, and decarboxylated rosin acids. Each solvent has at least one of: a) at least one OH group, b) at least one ester and / or ether group, and c) 10 or more carbon atoms. Contacting the substrate with the surface having the pre-applied solvent system, for the sulfonated block copolymer to be imparted from the substrate to the surface by being dissolved in the pre-applied solvent system, forming a coating layer on the surface. The coating layer causes reduction in a microbe concentration by at least 1 log10 CFU (colony forming units) within 120 minutes upon contact with the coating layer.

In a fifth aspect, a method for protecting a surface, comprising: providing a dry wipe having a substrate impregnated with a sufficient amount of a sulfonated block copolymer having an ion exchange capacity (IEC) of at least 0.5 meq/g. Pre-applying a solvent system onto the substrate to dissolve the sulfonated block copolymer. The solvent system contains a mixture of two or more solvents selected from the group consisting of organic solvents, inorganic solvents, terpene-based compounds, and decarboxylated rosin acids. Each solvent has at least one of: a) at least one OH group, b) at least one ester and / or ether group, and c) 10 or more carbon atoms. Contacting the substrate with the surface to be protected, for the sulfonated block copolymer dissolved in the pre-applied solvent system to be imparted from the substrate to the surface, forming a coating layer on the surface. The coating layer causes reduction in a microbe concentration by at least 1 log10 CFU (colony forming units) within 120 minutes upon contact with the coating layer.

### DETAILED DESCRIPTION

The following terms will be used throughout the specification.

"At least one of [a group such as A, B, and C]" or "any of [a group such as A, B, and C]" means a single member from the group, more than one member from the group, or a combination of members from the group. For example, at least one of A, B, and C includes, for example, A only, B only, or C only, as well as A and B, A and C, B and C; or A, B, and C, or any other all combinations of A, B, and C. A list of embodiments presented as "A, B, or C" is to be interpreted as including the embodiments, A only, B only, C only, "A or B," "A or C," "B or C," or "A, B, or C".

"Shear force" refers to an unaligned force acting on one part of a body in a specific direction and another part of the body in the opposite direction. The term shear force can be used in place or together with compression force or compressive force.

"Wipe" means a flexible sheet, a piece of fabric, a paper, a towel, or a sponge that can partially or fully absorb a fluid, for the removal or erase of a marking on a surface.

"Moist wipe" refers to a wipe substrate containing a liquid or gel applied thereto so that a sufficient amount of the liquid / gel is retained on or within the wipe substrate until its utilization by a user, for the wipe to be damp or slightly damp (and not dry) to the user upon touching.

"Wet wipe" refers to a wipe substrate with liquid retained on or within the substrate so the wipe feels wet to the user upon touching, or the wipe is covered or saturated with liquid.

"Microbes" refers to microorganisms including bacteria, archaea, fungi (yeasts and molds), algae, protozoa, and viruses, with microscopic size.

"Surface pH" refers to a pH on a contact surface of a material, e.g., fabric, plastic, etc., that results from surface bound moieties. The surface pH can be measured with commercial surface pH measuring instruments, e.g., SenTix^{™} Sur-electrode from WTW Scientific-Technical Institute GmbH, Weilheim, Germany.

"Solubility Parameter" or (δ) of a solvent or polymer, refers to the square root of the vaporization energy (ΔE) divided by its molar volume (V), as in the equation δ = (ΔE/V)^{1/2}. The more similar the solubility parameters of two substances, the higher will be the solubility between them and hence the expression "like dissolves like." Hansen established that the solubility parameter of a solvent or polymer is the result of the contribution of three types of interactions: dispersion forces (δ_{D}²), polar interactions (δ_{P}²) and hydrogen bonds (δ_{H}²) (Hansen, 2007; Hansen, 1967), with the total solubility (Hildebrand) parameter δT defined as: δ_{T} = (δ²_{D} + δ²_{P} + δ²_{H} )^{½}.

"Molecular weight" or Mw refers to the polystyrene equivalent molecular weight in g/mol of a polymer block or a block copolymer. Mw can be measured with gel permeation chromatography (GPC) using polystyrene calibration standards, such as is done according to ASTM 5296-19. The GPC detector can be an ultraviolet or refractive index detector or a combination thereof. The chromatograph is calibrated using commercially available polystyrene molecular weight standards. Mw of polymers measured using GPC so calibrated are polystyrene equivalent molecular weights or apparent molecular weights. Mw expressed herein is measured at the peak of the GPC trace and are commonly referred to as polystyrene equivalent "peak molecular weight," designated as Mₚ.

"Susceptible to sulfonation" refers to a polymer, polymer block, compound, monomer, oligomer, etc., being predisposed, or sensitive, or capable of reaction with sulfur containing compound, e.g., SO₃, H₂SO₄, etc., under conditions conventionally employed for sulfonation, wherein sulfonation is very likely to occur to obtain a sulfonated product. In embodiments, a polymer block "susceptible to sulfonation" upon sulfonation, the degree of sulfonation is at least 10 mol%, or at least 20 mol%, or at least 30, or at least 50 mol%, or at least 75 mol%, of the total polymer block, for the polymer block to have at least 10 mol%, or at least 20 mol%, or at least 30, or at least 50 mol%, or at least 75 mol% sulfonic acid or sulfonate ester functional groups.

"Resistant to sulfonation" means having little if any sulfonation of the respective block under conditions conventionally employed for sulfonation, with < 10 mol%, or < 8 mol%, or < 5 mol% sulfonic acid or sulfonate ester functional groups in the polymer block.

"Polystyrene content" or PSC of a block copolymer refers to the weight % of polymerized vinyl aromatic monomers, e.g., styrene in the block copolymer, calculated by dividing the sum of molecular weight of all vinyl aromatic units by total molecular weight of the block copolymer. PSC can be determined using any suitable methodology such as proton nuclear magnetic resonance (NMR).

"Sulfonated styrenic block polymer" or SSBC refers to a styrenic block copolymer precursor which contains sulfonic acid and / or sulfonate ester groups.

The disclosure relates to an antimicrobial wipe for protecting a surface, comprising a wipe substrate impregnated with a sufficient amount of an antimicrobial composition. The antimicrobial composition contains a sulfonated block copolymer and at least a solvent system. The sulfonated block copolymer has antimicrobial properties for killing at least 95% microbes within a pre-defined duration of contact with the sulfonated block copolymer. A coating layer obtained from the antimicrobial composition onto the surface is effective in killing microbes for longer duration and can be used in places prone to microbial contamination.

### (Wipe Substrate)

The wipe substrate can be any of a towelette, an absorbent sheet, a fabric, a sponge, a paper, an absorbent polymer etc., with sufficient absorption and holding capacity to retain the antimicrobial composition in the substrate, to protect targeted surfaces.

In embodiments, the wipe substrate is any of knit, woven, or nonwoven material, having a pre-determined size, e.g., a sheet or a towelette, which can be a single layer or multiple layers forming a laminate construct, with at least a surface with outer layer comprising fibers. The fibers can be natural, e.g., wool, silk, jute, hemp, cotton, linen, sisal, ramie, and the like; or synthetic, e.g., rayon, cellulose ester, polyvinyl derivatives, polyolefins, polyamides, polyesters, and the like.

In embodiments, the fibers include a combination of oleophilic fibers and hydrophilic fibers with a weight ratio of 10:90 to 90:10, or 30:70 to 70:30, or 40:60 to 60:40, or 50:50. Oleophilic refers to fibers that exhibit a static water contact angle of < 40°. Non-limiting examples of oleophilic fibers include polyurethane fibers, polypropylene fibers, polyethylene fibers, polyethylene terephthalate fibers, or mixtures thereof. Hydrophilic refers to fibers that exhibit a static water contact angle > 40°. Examples of hydrophilic fibers include but are not limited to cellulose fibers, rayon fibers, cotton fibers, polyamide fibers, polyacrylic acid fibers, or mixtures thereof.

The fibers can be distributed haphazardly or in random array, or substantially aligned. The fibers can be oriented or carded into fibrous webs. Thermoplastic fibers can be formed into thermocarded nonwoven webs, which can be spun bonded, i.e., spun out onto a flat surface and bonded / melted together by heat or chemical reactions. The fibers can be thermally or adhesively bonded together.

In embodiments, the wipe substrate is a multi-layered structure having some of the individual layers, or only the outermost layer is impregnated with the antimicrobial composition providing the antimicrobial properties.

In embodiments, the wipe substrate is a sponge, e.g., a porous structure, a foam with foam matrix for impregnation of the antimicrobial composition. The sponge can be any of a natural animal sponge, a natural plant sponge, or a synthetic sponge comprising any of polyester, polyurethane, cellulose, or mixtures thereof.

In embodiments, the wipe substrate is of the form as disclosed in US Patent Application Publication No. 2002/0192268A1. The wipe substrate is in the form of a substrate having a plurality of "micro-pockets" with a ratio of depth to width of at least 1:2, and with the mini pockets having a size (width) of 100 - 400 µm, or > 150 µm, or > 200 µm, or < 500 µm. In embodiments, the mini-pockets have a length / width of 0.1 - 100 mm, and a depth of 0.05 - 10 mm. The mini pockets can be formed on a non-woven substrate by ring rolling or heated toothed rotating cylinder on the substrate, prior to the application and loading of an antimicrobial composition onto the substrate. The micro-pockets allow for controlled release of the antimicrobial composition "contained" in the mini pockets based on a simple lateral wiping action.

In embodiments, the wipe substrate has a water absorption rate of > 8 g, > 9 g, > 10 g, or > 10.5 g, or as high as 100 g, or 500 g water per 1 g of wipe substrate. In embodiments, the wipe substrate has an oil absorption rate of > 9 g, or > 10 g, or > 12 g oil per 1 m² of fibrous substrate (area).

In embodiments, the wipe substrate has a wide mass area, e.g., a substrate mass area of at least 20 g/m² (or gsm), or > 30 gsm, or > 40 gsm, or > 60 gsm, or < 250 gsm.

In embodiments, the wipe substrate further comprises additional components for functions such as absorbency, liquid repellence, resilience, stretch, softness, strength, flame retardancy, washability, cushioning, etc. In embodiments, the wipe substrate in the form fabric includes a slip additive to enhance the softness of the wipe substrate.

### (Sulfonated Block Copolymer)

Sulfonated block copolymer refers to polymers having a sulfonate group, e.g., -SO₃, either in the acid form (e.g., -SO₃H, sulfonic acid) or a salt form (e.g., -SO₃Na). The term "sulfonated block copolymer" also covers sulfonate containing polymers, e.g., polystyrene sulfonate.

In embodiments, the sulfonated block copolymer is characterized as being sufficiently sulfonated, meaning having at least 10 mol% of sulfonic acid or sulfonate ester functional groups based on total mol of the number of monomer units or polymer blocks to be sulfonated ("degree of sulfonation"). In embodiments, the sulfonated block copolymer has a degree of sulfonation of at least 10 mol%, or > 15, or > 20, or > 25, or > 30, or > 40, or > 50, or > 60, or > 70, or > 80, or > 90, or > 99 mol%. The degree of sulfonation can be calculated by NMR or ion exchange capacity (IEC). In embodiments, the sulfonated block copolymer has an ion exchange capacity (IEC) of at least 0.5, or > 0.75, or > 1.0, or > 1.5, or > 2.0, or > 2.5, or < 5.0 or 0.5 - 3.5, or 0.5 - 2.6 meq/g. In embodiments, the layer has a surface pH of < 5, or < 3.0, or < 2.5, or < 2.25, or < 2.0, or < 1.80.

In embodiments, the sulfonated block copolymer is a sulfonated styrenic block copolymer (SSBC) obtained by sulfonation of a styrenic block copolymer (SBC) precursor which is any of linear, branched, or radial block copolymer having at least one end block A and at least one interior block B. The SSBC has at least a sulfonate group, e.g., -SO₃, either in an acid form (e.g., -SO₃H, sulfonic acid) or a salt form (e.g., -SO₃Na). The sulfonate group can be in the form of metal salt, ammonium salt, or amine salt.

In embodiments, SBC precursor is prepared by anionic polymerization using techniques known in the art. Other methods, such as cationic polymerization, can also be employed. The anionic polymerization initiator is generally an organometallic compound, such as, an organolithium compound, e.g., ethyl-, propyl-, isopropyl-, n-butyl-, sec-butyl-, tert-butyl-, phenyl-, hexylbiphenyl-, hexamethylenedi-, butadieneyl-, isopreneyl-, 1,1-diphenylhexyllithium, or polystyryllithium. An amount of initiator needed is calculated based on the molecular weight to be achieved, generally from 0.002 to 5 wt.%, based on amount of monomers to be polymerized. Suitable solvent for the polymerization includes aliphatic, cycloaliphatic, or aromatic hydrocarbons having from 4 to 12 carbon atoms, such as pentane, hexane, heptane, cyclopentane, cyclohexane, methylcyclohexane, decalin, isooctane, benzene, alkylbenzenes, such as toluene, xylene or ethylbenzene, and mixtures thereof. Polymer chain termination can be achieved by quenching with a proton donor or a compound having a leaving group that can be displaced by the carbanionic polymer chain.

If desired, a Lewis base additive, which affects polymerization parameters can also be employed. Examples of Lewis bases include dimethyl ether, diethyl ether, ethylene glycol dimethyl ether, 1,2-diethoxypropane, diethylene glycol dimethyl ether, tetrahydrofuran, tetrahydrofurfuryl ethers, such as tetrahydrofurfuryl methyl ether, and tertiary amines. The additives can influence the extent of 1,2-addition of the conjugated diene, and therefore the vinyl group content in the respective block.

In embodiments, the SBC precursor has a general configuration of: A-B-A, (A-B)ₙ(A), (A-B-A)ₙ, (A-B-A)ₙX, (A-B)ₙX, A-D-B, A-B-D, A-D-B-D-A, A-B-D-B-A, (A-D-B)ₙA, (A-B-D)ₙA (A-D-B)ₙX, (A-B-D)ₙX, (A-D-B-D-A)ₙX, (A-B-D-B-A)ₙX or mixtures thereof; where n is an integer from 2 to 30; and X is a residue of a coupling agent. Each block A and D is resistant to sulfonation, and each block B is susceptible to sulfonation.

In embodiments, the coupling agent X includes bi- or polyfunctional compounds, for example divinylbenzene, halides of aliphatic or araliphatic hydrocarbons, such as 1,2-dibromoethane, bis(chloromethyl)benzene, or silicon tetrachloride, dialkyl- or diarylsilicon dichloride, alkyl- or arylsilicon trichloride, tin tetrachloride, alkylsilicon methoxides, alkyl silicon ethoxides, polyfunctional aldehydes, such as terephthalic dialdehyde, ketones, esters, anhydrides, or epoxides. In embodiments, the coupling agent is selected from methyltrimethoxysilane (MTMS), methyltriethoxysilane (MTES), tetramethoxysilane (TMOS), dimethyladipate, gamma-glycidoxypropyltrimethoxy silane, and mixtures thereof.

In embodiments, the block A has at least one compound selected from polymerized (i) para-substituted styrene, (ii) ethylene, (iii) alpha olefins of 3 to 18 carbon atoms; (iv) 1,3-cyclodiene, (v) conjugated dienes having a vinyl content of < 35 mol% prior to hydrogenation, (vi) acrylic esters, (vii) methacrylic esters, and (viii) mixtures thereof. If the block A is a polymer of 1,3-cyclodiene or conjugated diene monomer, the block A will be hydrogenated subsequent to preparation of the SBC and before sulfonation of the SBC precursor. In embodiments, the block A contains up to 15 wt.% of vinyl aromatic monomers such as those present in the block B.

In embodiments, the block A is derived from polymerized para-substituted styrene monomers selected from the group consisting of para-methylstyrene, para-ethylstyrene, para-n-propylstyrene, para-iso-propylstyrene, para-n-butylstyrene, para-sec-butylstyrene, para-iso-butylstyrene, para-t-butylstyrene, isomers of para-decylstyrene, isomers of para-dodecylstyrene, and mixtures thereof.

In embodiments, the block A has a Mₚ of 1 - 60, or 2 - 50, or 5 - 45, or 8 - 40, or 10 - 35, or > 1.5, or < 50 kg/mol. In embodiments, the block A constitutes from 1- 80, or 5 - 75, or 10 - 70, or 15 - 65, or 20 - 60, or 25 - 55, or 30 - 50, or > 10, or < 75 wt.%, based on total weight of the SBC precursor.

In embodiments, the block B is derived from polymerized vinyl aromatic monomers selected from the group consisting of unsubstituted styrene, ortho-substituted styrene, meta-substituted styrene, alpha-methylstyrene, 1,1-diphenylethylene, 1,2-diphenylethylene, and mixtures thereof. In embodiments, the block B has a mixture of the vinyl aromatic monomer and hydrogenated conjugated dienes, such as butadiene or isoprene, having a vinyl content, prior to hydrogenation, of 2 - 40, or 5 - 38, or 8 - 35, or 10 - 30, or > 5, or < 40 wt.%, based on total weight of polymerized conjugated diene monomers in the block B.

In embodiments, the block B has a Mₚ of 10 - 300, or 20 - 250, or 30 - 200, or 40 - 150, or 50 - 100, or 60 - 90, or > 15, or < 150 kg/mol. In embodiments, the block B constitutes from 10 - 80, or 15 - 75, or 20 - 70, or 25 - 65, or 30 - 55, or > 10, or < 75 wt.%, based on total weight of the SBC precursor. In embodiments, the block B has from 0 - 25, or 2 - 20, or 5 - 15 wt.%, of the para-substituted styrene monomers such as those present in the block A.

In embodiments, the block D is derived from a polymer or copolymer of a conjugated diene monomer selected from the group consisting of isoprene, 1,3-butadiene, 2,3-dimethyl-1,3-butadiene, 1-phenyl-1,3-butadiene, 1,3-pentadiene, 1,3-hexadiene, 3-butyl-1,3-octadiene, farnesene, myrcene, piperylene, cyclohexadiene, and mixtures thereof. In embodiments, the block D has a Mₚ of 1 - 60, or 2 - 50, or 5 - 45, or 8 - 40, or 10 - 35, or 15 - 30, or > 1.5, or < 50 kg/mol. In embodiments, the block D constitutes from 10 - 80, or 15 - 75, or 20 - 70, or 25 - 65, > 10, or < 75 wt.%, based on total weight of the SBC precursor.

In embodiments, prior to hydrogenation and sulfonation, the block D has a vinyl content of > 80, or > 85, or > 90, or > 95, or > 98, or > 99, or > 99.5, or 50 - 95, or 60 - 90, or 70 - 95 wt.%, based on total weight of the polymerized conjugated diene monomers in the block D.

In embodiments, the block D has a hydrogenation level of 60 - 99%, or 65 - 95%, or 70 - 90%, or 75 - 99%, or > 75%, or 85%, or > 95%, or > 98%.

In embodiments, each block A and B independently has a hydrogenation level of 0 - 20%, or 2 - 18%, or 4 - 15%, or > 10%, or > 15%, or < 20%. A suitable catalyst based on nickel, cobalt or titanium can be used in the hydrogenation step.

The SBC precursor is sulfonated to provide the corresponding SSBC. Sulfonation occurs at the phenyl ring of polymerized styrene units in the block B, predominantly para to the phenyl carbon atom bonded to the polymer backbone. In embodiments, the block B has a degree of sulfonation of 10 - 100, or 15 - 95, or 20 - 90, or 25 - 85, or 30 - 80, or 35 - 75, or 40 - 70, or > 15, or < 85 mol%, based on total mol of the block B.

In embodiments, the sulfonated block copolymer is a midblock-sulfonated triblock copolymer, or a midblock-sulfonated pentablock copolymer, e.g., a poly(p-tert-butylstyrene-b- styrenesulfonate-b-p-tert-butylstyrene), or a poly[tert-butylstyrene-b-(ethylene-alt-propylene)-b-(styrenesulfonate)-b-(ethylene-alt-propylene)-b-tert-butylstyrene.

In embodiments, the sulfonated block copolymer has a Mₚ of 25 - 500, or 30 - 450, or 350 - 400, or 40 - 350, or 45 - 300, or 50 - 250, or > 35, or < 350 kg/mol.

In embodiments, the sulfonated block copolymer has a glass transition temperature (T_{g}) of 80 - 180°C, or 85 - 160°C, or 90 - 150°C, or 100 - 140°C, or > 90°C, or < 210°C, measured by Dynamic Mechanical Analysis (DMA), according to ASTM 4065.

The sulfonated block copolymer in embodiments is as disclosed in Patent Publication Nos. US9861941, US8263713, US8445631, US8012539, US8377514, US8377515, US7737224, US8383735, US7919565, US8003733, US8058353, US7981970, US8329827, US8084546, US8383735, and US10202494.

The antimicrobial and mechanical properties of the sulfonated block copolymer can be varied and controlled by varying the amount of sulfonation, the degree of neutralization of the sulfonic acid groups to the sulfonated salts, as well as controlling the location of the sulfonated group(s) in the polymer. In embodiments and depending on the applications, e.g., one with the need for water dispersity / solubility, or at the other spectrum, one with the need for sufficient durability with constant wiping with water based cleaners, the sulfonated block copolymer can be selectively sulfonated for desired water dispersity properties or mechanical properties, e.g., having the sulfonic acid functional groups attached to the inner blocks or middle blocks, or in the outer blocks of a sulfonated block copolymer, as in US Patent No. US8084546. If the outer (hard) blocks are sulfonated, upon exposure to water, hydration of the hard domains may result in plasticization of those domains and softening, allowing dispersion or solubility.

Depending on the applications and the desired properties, the sulfonated block copolymer can be modified (or functionalized). In embodiments, the sulfonated block copolymer is neutralized with any of various metal counterions, including alkali, alkaline earth, and transition metals, with at least 10% of the sulfonic acid groups being neutralized. In embodiments, the sulfonated block copolymer is neutralized with inorganic or organic cationic salts, e.g., those based on ammonium, phosphonium, pyridinium, sulfonium, and the like. Salts can be monomeric, oligomeric, or polymeric. In embodiments, the sulfonated block copolymer is neutralized with various primary, secondary, or tertiary amine-containing molecules, with > 10% of the sulfonic acid or sulfonate ester functional groups being neutralized.

In embodiments, the monomer or the block containing amine functionality or phosphine functionality can be neutralized with acids or proton donors, creating quaternary ammonium or phosphonium salts. In other embodiments, the sulfonated block copolymer containing tertiary amine is reacted with alkylhalides to form functional groups, e.g., quaternized salts. In some embodiments, the sulfonated block copolymer can contain both cationic and anionic functionality to form so-called zwitterionic polymers.

In embodiments, the sulfonic acid or sulfonate functional group is modified by reaction with an effective amount of polyoxyalkyleneamine having molecular weights from 140 to 10,000. Amine-containing neutralizing agents can be mono-functional or multifunctional; monomeric, oligomeric, or polymeric. In alternative embodiments, the sulfonated block copolymer is modified with alternative anionic functionalities, such as phosphonic acid or acrylic and alkyl acrylic acids.

In embodiments, amine containing polymers are used for the modification of the sulfonated block copolymers, forming members of a class of materials termed coaservates. In examples, the neutralizing agent is a polymeric amine, e.g., polymers containing benzylamine functionality. Examples include homopolymers and copolymers of 4-dimethylaminostyrene which has been described in US Patent 9,849,450, incorporated herein by referene. In embodiments, the neutralizing agents are selected from polymers containing vinylbenzylamine functionality, e.g., polymers synthesized from poly-p-methylstyrene containing block copolymers via a bromination-amination strategy, or by direct anionic polymerization of amine containing styrenic monomers. Examples of amine functionalities for functionalization include but are not limited to p-vinylbenzyldimethylamine (BDMA), p-vinylbenzylpyrrolidine (VBPyr), p-vinylbenzyl-bis(2-methoxyethyl)amine (VBDEM), p-vinylbenzylpiperazine (VBMPip), and p-vinylbenzyldiphenylamine (VBDPA). In embodiments, corresponding phosphorus containing polymers can also be used for the functionalization of the sulfonated block copolymers.

Other sulfonated block copolymer can be selected from sulfonated polystyrene-polyisoprene-polystyrene sulfonated in the center segment, sulfonated t-butylstyrene / isoprene random copolymer with C=C sites in their backbone, sulfonated SBR (styrene butadiene rubber) as disclosed in US 6,110,616, and mixtures thereof. In embodiments, the sulfonated block copolymer is a water dispersible BAB triblock, with B being a hydrophobic block such as alkyl or (if it is sulfonated, it becomes hydrophilic) poly(t-butyl styrene) and A being a hydrophilic block such as sulfonated poly(vinyl toluene) as disclosed in US 4,505,827. In embodiments, the sulfonated block copolymer is a water-soluble polymer, a sulfonated diblock polymer of t-butyl styrene / styrene, or a sulfonated triblock polymer of t-butyl styrene -styrene - t-butyl styrene as disclosed in US 4,492,785.

In embodiments, the sulfonated block copolymer is a water-soluble polymer, a sulfonated diblock polymer of t-butyl styrene / styrene, or a sulfonated triblock polymer of t-butyl styrene -styrene - t-butyl styrene as in US 4,492,785.

In embodiments, the antimicrobial composition comprises combinations of two or more sulfonated block copolymers with each having different degree of sulfonation and / or IECs.

In embodiments, the sulfonated block copolymer is used in amounts of 0.1 - 50, or 0.5 - 20, or 0.1 - 10, or 1- 25, or 5 - 20 wt.%, based on total weight of the antimicrobial composition.

### (Solvent System)

The sulfonated block copolymer is dissolved in a solvent system, forming the antimicrobial composition for impregnating the wipe substrate. In embodiments, the solvent system contains a solvent selected from the group consisting of organic solvents, inorganic solvents, terpene-based solvents, decarboxylated rosin acids, and mixtures thereof. In embodiments, the solvent system contains a mixture of at least two or more solvents. By dissolved, it means that the sulfonated block copolymer is partially dissolved or fully dissolved in the solvent system. Partially dissolved means > 40% and < 100% of the sulfonated block copolymer is dissolved in the solvent, or 50 - 99%, or 60 - 95%, or > 70% but < 100%.

In embodiments, the solvent is an organic solvent selected from acetone, acetyl acetone, acetic acid, acetonitrile, benzonitrile, 2-butanone, t-butyl alcohol, benzyl alcohol, cyclohexane, cyclohexanol, cyclohexanone, dichloromethane, 1,2-dichloroethane, diethylene glycol, diethyl ether, diglyme (diethylene glycol dimethyl ether), 1,2-dimethoxyethane (glyme, DME), 1,4-dioxane, methanol, ethanol, methyl acetate, ethyl acetate, ethyl acetoacetate, butyl acetate, ethylene glycol, glycerin, heptane, hexamethylphosphoramide (HMPA), hexamethylphosphoroustriamide (HMPT), hexane, methyl t-butyl ether (MTBE), dimethylsulfoxide (DMSO), methyl ethyl ketone (MEK), dimethylformamide (DMF), methyl propyl ketone (MPK), dimethylphthalate, nitromethane, pentane, petroleum ether, pyridine, toluene, aniline, triethyl amine, diethylamine, o-xylene, m-xylene, p-xylene, naphtha, di-n-butylphthalate, 3-pentanone, chloroform, bis(2-methoxyethyl) ether, ethyl benzoate, tetrahydrofuran(THF), anisole, chlorobenzene, N,N-dimethylaniline, carbon disulfide, carbon tetrachloride, ethylene glycol mono hexyl ether (EGMHE), 1-undecanol, 1-pentanol, tert-butyl methyl ether, 2-ethyl butyl acetate, butyl lactate, N-dodecane, butanol, ethyl lactate, 1-decanol, isopropyl myristate, 1-propanol, 2-propanol, isododecane, ethyl acetate, decanal, ethyl glycol acetate, 2-ethyl hexanoic acid, neopentyl glycol, and mixtures thereof. In embodiments, the organic solvent is selected from the group of C₁ - C₆ alkanols, C₁ - C₆ diols, C₁ - C₆ alkyl ethers of alkylene glycols and polyalkylene glycols, and mixtures thereof.

In embodiments, the solvent is an inorganic solvent selected from water, ammonia, and mixtures thereof.

In embodiments, the solvent is a terpene-based compound selected from d-limonene, alpha-pinene, beta-pinene, 1-methyl-4-isopropylene-1-cyclohexane, and mixtures thereof.

In embodiments, the solvent is a decarboxylated rosin acid (DCR) in the form of a liquid, which is either a crude DCR, a distilled or purified DCR (> 90% purity), or mixtures thereof, as disclosed in US Patent Publication No. US20220195326A1.

In embodiments, the solvent is selected from solvents having a relative polarity of 0.005 - 0.65, or 0.006 - 0.64, or 0.007 - 0.63, or 0.008 - 0.62, or 0.009 - 0.61.

In embodiments, the solvent is characterized to have at least one of: at least one OH group, at least one ester and / or ether group, or 10 or more carbon atoms.

In embodiments, the solvent system contains a mixture of two solvents different from each other, in a weight ratio of 10:90 to 90:10, or 20:80 to 80:20, or 70:30 to 30:70, or 60:40 to 40:60. Examples of pair of solvents include: ethyl acetate and 1-propanol, tert-butyl methyl ether and 1-propanol, 2-ethyl butyl acetate and 1-propanol, butyl lactate and butanol, 1-decanol and butanol, 1-undecanol and isopropyl myristate, 1-propanol and isopropyl myristate, decanal and isopropyl myristate, 1-undecanol and butanol, 2-ethyl butyl acetate and 1-propanol, ethyl lactate and butanol, isododecane and butanol, decanal and butanol, 1-undecanol and isododecane, decanal and isododecane, n-dodecane and butanol, ethyl glycol acetate and 1-propanol, ethyl glycol acetate and butanol, 1-pentanol and isopropyl myristate, 1-undecanol and 1-pentanol, 2-ethyl hexanoic acid and ethyl acetate, EGMHE and 1-propanol, EGMHE and butanol, EGMHE and ethyl lactate, EGMHE and ethyl glycol acetate, EGMHE and 1-pentanol, EGMHE and 1-decanol, EGMHE and isododecane, EGMHE and decanal, isododecane and 1-pentanol, 2-ethyl hexanoic acid and EGMHE, 2-ethyl hexanoic acid and ethyl acetate, EGMHE and ethanol, isododecane and ethanol, ethyl glycol acetate and ethanol, and neopentyl glycol and ethanol.

In embodiments, the solvent system contains more than one solvent, wherein each solvent in the solvent system is different from each other, e.g., two different solvents, or three different solvents, etc.

In embodiments, the solvent system comprises a mixture of solvents, with HSP (Hildebrand Solubility Parameter) δD ranging from 15 - 16, or 15.05 - 15.90, or 15.10 - 15.80, or 15.15 - 15.70, or 15.20 - 15.60, or 15.25 - 15.55, a δP ranging from 1 - 7, or 1.2 - 6.8, or 1.4 - 6.5, or 1.5 - 6.0, or 1.6 - 5.6; and a δH ranging from 2 - 9, or 2.1 - 8.5, or 2.2 - 8.0, or 2.2 - 7.5, or 2.3 - 7, or 2.4 - 6.8.

In embodiments, the solvent system containing a single solvent, or a mixture of solvents, with each solvent independently having a δD of 15 - 16, or 15.05 - 15.90, or 15.10 - 15.80, or 15.15 - 15.70, or 15.20 - 15.60, or 15.25 - 15.55; a δP of 1 - 7, or 1.2 - 6.8, or 1.4 - 6.5, or 1.5 - 6.0, or 1.6 - 5.6; and a δH of 2 - 9, or 2.1 - 8.5, or 2.2 - 8.0, or 2.2 - 7.5, or 2.3 - 7, or 2.4 - 6.8.

In embodiments, the solvent system has a total solubility parameter (*δ_{T}*) of ≥ 14.5, or ≥ 15, or ≥ 15.5, or 15 - 19, or 15.2 - 18.8, or 15.5 - 18.6, or 15.7 - 18.5, or 15.8 - 18.2.

In embodiments, if the solvent is not water itself, the solvent system can be further diluted with water for an aqueous system, at a weight ratio of the solvent system to water ranging from 10:90 to 90:10, or 20:80 to 80:20, or 70:30 to 30:70, or 60:40 to 40:60, for a viscosity of < 500 centipoise (cps), or < 200 cps, or < 100 cps, or < 50 cps. The lower viscosity solvent formulation allows the antimicrobial composition to wick quickly into a stack of dry precut wipes, for the antimicrobial composition to subsequently transfer from the wipe substrate to the surface intended to be contacted / coated with the antimicrobial composition (for a coating layer).

In embodiments, the solvent system is used in an amount of 50 - 99.90, or 80 - 99.50, or 90 - 99.90, or 75 - 99, or 80 - 95 wt.%, based on total weight of the antimicrobial composition.

### (Optional Additives)

The antimicrobial composition further comprises at least one additive selected from the group consisting of activators, curing agents, neutralizing agents, thickeners, coalescing agents, slip agents, release agents, surfactants, antioxidants, antimicrobial agents, antiozonants, color change pH indicators, luminescent additives (e.g., phosphorescent and fluorescence), plasticizers, tackifiers, film forming additives, dyes, pigments, UV stabilizers, UV absorbers, catalysts, fillers, other polymers, fibers, flame retardants, viscosity modifiers, wetting agents, deaerators, toughening agents, adhesion promoters, colorants, heat stabilizers, lubricants, flow modifiers, drip retardants, antiblocking agents, antistatic agents, processing aids, stress-relief additives, enzymes, color stabilizers, and mixtures thereof.

Non-limiting examples of antimicrobial agents other than the sulfonated block copolymer include quaternary ammonium group containing polymers, phosphonium group containing polymers, chitosan and other naturally occurring antimicrobial polymers, ionexchange resins, silver, copper, zinc, titanium and their oxides, and mixtures thereof. In embodiments, the antimicrobial composition further comprises components known for or having the effect of killing microbes upon contact (e.g., in < 1 min., < 45 sec., < 30 sec.), Examples such components include but not limited to hypochlorous acid, isopropyl alcohol (IPA), chlorhexidine, hydrogen peroxide, etc., allowing the antimicrobial wipe to have instant antimicrobial effect, as well as residual or long-term efficacy.

In embodiments, the addition of luminescent additives such as phosphorescent and fluorescence would help or enable the antimicrobial composition to illuminate, or optical brighteners additives that illuminate under a special UV or black light tracer, allowing for physical inspections to verify that the wipe, and subsequently the intended surfaces are protected with the antimicrobial composition for antimicrobial / self-disinfecting effects.

In embodiments, the antimicrobial composition contains a color-changing ink / dye that is sensitive to the concentration and / or presence of the antimicrobial composition on the surface as an indicator as whether more antimicrobial composition should be impregnated into the wipe, or the surface to be protected should be wiped again. In embodiments, the antimicrobial composition contains a color-changing ink / dye, such that at the moment of contact with moisture from a solution or a solvent, the wipe changes color producing a visual indication that the wipe substrate is ready to be applied onto the surface, e.g., with shear force as in a wiping or coating motion to form a coating layer on the surface.

Surfactants to be incorporated into the antimicrobial composition can be selected from anionic, nonionic, cationic, and zwitterionic, depending on the solvent components employed. Non-limiting examples of surfactants include nonionic ethylene oxide (EO) containing surfactants, carboxylates, primary, secondary and tertiary monoamines with C₁₈ alkyl or alkenyl chains, ethoxylated alkylamines, bataines, imidazolines, propinates, and mixtures thereof.

In embodiments, the antimicrobial composition includes an adhesion promoter / binder selected from the group consisting of acrylate emulsions, butadiene-styrene emulsions, ethylene vinyl acetate emulsions, and acrylonitrile-butadiene emulsions.

In embodiments, the additive (if present) added in the antimicrobial composition is in amounts of 0.1 - 10, or 0.5 - 5, or 1 - 10, or 0.1 - 5, or 0.5 - 5 wt.%, based on total weight of the antimicrobial composition.

### (Methods for Constructing Antimicrobial Wipe)

In embodiments, the antimicrobial composition is obtained by partially or fully dissolving a sufficient amount of the sulfonated block copolymer and optional additives in a solvent system. The solvent system can be heated to a sufficient temperature, before or after the addition of the sulfonated block copolymer, to make the antimicrobial composition uniform and homogenous. In embodiments, the solvent system is heated at a temperature of 35 - 80°C, or 40 - 75°C, or 45 - 70°C, or 35 - 60°C.

The antimicrobial wipe can be constructed by incorporating a sufficient amount of antimicrobial composition into the wipe substrate (or a wet wipe having a substrate) by any of impregnation, coating, immersing, dipping, sinking, drench, covering, rinsing, wetting, absorbing, and the like, for a sufficient period so that the wipe substrate holds or retains sufficient amount of the antimicrobial composition for cleaning / coating a target surface for killing 99 % microbes.

In embodiments, a coating layer containing the antimicrobial composition is formed on the wipe substrate by electrospinning (e-spun), generating multiple layers of nanoscale to microscale antimicrobial fibers with disinfecting properties. In embodiments, the electrospinning is at a rate of 1 - 30 grams antimicrobial composition per square meter per mil thickness, or 2 - 10 g/m², or at least 3 g/m² per mil thickness of the wipe. In embodiments, the electrospun antimicrobial microfibers or nanofibers are interweaved with other fibers, e.g., microfibers, submicron fibers and nanofibers from other different polymers, forming the antimicrobial wipe.

In embodiments, the antimicrobial composition is applied by spraying with the partially / fully dissolved sulfonated block copolymer with a sprayer forming a coating layer on the wipe substrate, or by dipping the wipe substrate into the solution until the wipe substrate is sufficiently saturated with the solution, to form a gel on the wipe substrate surface, or for the antimicrobial composition to fully or almost fully saturate the wipe substrate for a pre-saturated (wet) wipe.

In embodiments with the wipe substrate containing mini-pockets, the antimicrobial composition is "loaded" into the mini-pockets by methods including immersion, squeegeeing, spraying, dot matrix deposition, slot coating, printing, filling, dipping, and individual ingredient imprinting on selected regions of the substrate laterally or at different depths of the substrate.

In embodiments, the antimicrobial wipe in the dry form is prepared by first embedding / depositing / impregnating the sulfonated block copolymer (as a solid, e.g., powder) into a wipe substrate (or a dry wipe having a substrate) which is then contacted or moistened with a solvent system that subsequently partially or fully dissolves the sulfonated block copolymer. Such wipe when used on a target surface forms a continuous or discontinuous coating layer for killing microbes.

Depending on the wipe substrate material and amount of the sulfonated block copolymer in the solvent system, a sufficient amount of the antimicrobial composition is applied such that the wipe substrate can be dry, moist, or wet. In embodiments, the sufficient amount of the antimicrobial composition is > 1, or > 2, or > 5, or > 10, or > 50, 1 - 10 or up to 100 wt. %, based on total weight of the wipe substrate.

In embodiments, at least 50% or > 60%, or > 70%, or 90%, or up to 100% surface of the antimicrobial wipe is covered with the antimicrobial composition.

In embodiments, a sufficient amount of the antimicrobial composition is applied or impregnated into the wipe substrate such that when used for wiping, an amount is released or imparted onto a target surface to have a coating layer thickness of > 1 µm, or > 5 µm, or > 10 µm, or > 20 µm, or > 50 µm, or > 100 µm, or > 500 µm, or < 10,000 µm.

In embodiments, the antimicrobial wipe is made available as a stack of multilayers of antimicrobial wipes, e.g., 2, or 5, or 10 or 20 or 50 layers etc. Wipes are preferably stored or sealed in a suitable container, e.g., pouches, bags, tubs, etc., to maintain the desired level of antimicrobial composition content over time.

### (Methods for Cleaning / Wiping / Deposition of Antimicrobial Composition)

A surface for cleaning / protecting with the antimicrobial wipe can be any of a soft surface, e.g., fabric, curtains, pillows, upholstery, bedspreads, etc., or a hard surface, e.g., counters, tables, doorknobs, keyboards, light switches, etc., surfaces in food service industry, public buildings, or medical facilities, etc. The surface can be any material capable of supporting the growth of microorganisms. The surface can be part of a relatively durable object, or a disposable object.

When a moist (damp, or slightly wet) antimicrobial wipe is in contact with the surface for cleaning or protection by shear force or compressive force, i.e., wiping once, or with back and forth motion, or by pressing motion, the antimicrobial wipe forms a thin continuous or discontinuous coating layer on the surface by transferring, or imparting, or leaving some or substantially all of the antimicrobial composition from the wipe substrate onto the surface as a coating layer. The coating layer is then allowed to evaporate the solvent system. In embodiments, > 10%, or > 20%, or > 40%, or > 60%, or > 80% of the antimicrobial composition is imparted from the moist wipe substrate onto the surface to form the coating layer.

In embodiments, the shear force is unidirectional, with the wiping being in one direction (no need to re-tort). In embodiments, the shear force is perpendicular to the surface to be wiped or cleaned by pressing down the wipe against the surface.

In embodiments, a solvent system is first applied on a surface to be wiped to wet the surface, then a dry wipe substrate is applied onto the wet surface, for the antimicrobial composition to be dissolved partially or fully, thus forming a coating layer on the surface.

In one embodiment, a dry antimicrobial wipe (pre-saturated with sulfonated block copolymer) is first placed on a surface, then a solvent system is applied onto the wipe substrate by spraying or other means, the soaked wipe is left for a sufficient period for the solvent to soften or dissolves the sulfonated block copolymer on the dry antimicrobial wipe. In another embodiment, a dry wipe is first sprayed with a solvent system to partially or fully dissolve the sulfonated block copolymer impregnated onto the wipe, then the soaked wipe is placed onto the surface. In either embodiment, some or most of the sulfonated block copolymer is transferred from the wipe substrate into the solvent system on the surface forming a coating layer.

In embodiments (wet, damp, or dry wipes), at least 10%, or > 20%, or > 40%, or > 60%, or > 80% of the sulfonated block copolymer is imparted from the wipe substrate onto the surface. Subsequently, the wipe substrate is removed, leaving the sulfonated block copolymer on the surface forming a coating layer after the solvent system evaporates.

The drying / hardening time depends on the sulfonated block copolymer employed in the antimicrobial wipe, the solvent system used, and the amount or thickness of the coating layer being spread on or applied on the surface. In embodiments, the drying time is < 5 min., or < 10 min., or < 1 min. or < 30 sec., or < 20 sec.

In embodiments, the thickness of the coating layer as formed on the surface - whether via a dry, damp, or wet wipe, is > 1 µm, or > 5 µm, or > 10 µm, or > 20 µm, or > 50 µm, or > 100 µm, or > 500 µm, or < 10,000 µm.

After a coating layer is formed with the antimicrobial wipe, the coating layer is found effective in destroying / inactivating at least 99.9% (3 log₁₀ CFU), or at least 99% (2 log₁₀ CFU), or at least 95%, or at least 90% (1 log₁₀ CFU) of microbes in < 30 min., or < 60 min., or < 120 min., for microbes include but are not limited to MRSA (Methicillin-resistant Staphylococcus aureus), vancomycin-resistant Enterococcus faecium, X-MulV, PI-3, SARS-CoV-2, carbapenem-resistant Acinetobacter baumannii, and influenza A virus. The coating layer containing the antimicrobial composition remains effective in killing microbes even after 4 hrs., or > 12 hrs., or > 24 hrs., or > 48 hrs. Depending on the solvent(s) applied, in embodiments, the coating layer as formed also provides immediate point-in-time kill (e.g., in < 1 min., or < 45 sec., or < 30 sec.) in addition to the supplemental residual antimicrobial effects.

### (End-Use Applications)

The antimicrobial wipe can be in various forms, including towelettes, absorbent sheets, sponges, coated or impregnated with an antimicrobial composition. The antimicrobial wipe can also be in the form of textured substrates with mini-pockets, or rupturable mini-pouches / pockets for controllably release of the antimicrobial composition. After application of the antimicrobial composition in gel, solution, or dispersion form to form a coating layer, can be dried forming a protective coating layer.

The antimicrobial wipe is preferably used on inanimate soft or hard surfaces for coating, particularly for controlling viruses, bacteria, and other harmful germs, and optionally cleaning. The surface can be in a home, commercial facilities, medical facilities, public buildings, transportation, etc. which is prone to microbial contamination.

### (Examples)

The following examples are intended to be non-limiting.

The components used in the examples include:
Sulfonated block copolymers SSBC-1, and SSBC-2: A sulfonated penta block copolymer of the structure poly[tert-butylstyrene-b-(ethylene-alt-propylene)-b-(styrene-co-styrene¬sulfonate)-b-(ethylene-alt-propylene)-tert-butylstyrene] (tBS-EP-sPS-EP-tBS) having properties shown in Table 1 are used for some of the examples.

**Table 1**

| Polymer | IEC (meg/g) | Degree of sulfonation (mol%) | MW (kg/mol) |
|---|---|---|---|
| SSBC-1 | 1.0 | 26 | 78 |
| SSBC-2 | 2.0 | 52 | 78 |

### (Example 1)

SSBC1 and SSBC-2 were separately mixed with 1:1 mixture of toluene and 1-propanol to obtain antimicrobial compositions. Film samples obtained from these antimicrobial compositions were subjected to abrasion testing of 2200 cycles in the presence of 3 common disinfectants: 1) 70% ethanol, benzalkonium chloride, and quaternary ammonia.

After abrasion testing, the film was cut into strips of 2" by 4" each. Tests were conducted to evaluate antimicrobial efficacy & the long-lasting antiviral properties of sulfonated copolymer film samples with exposure to SARS-CoV-2 virus. In the test, a SARS-CoV-2 vial (1.3×10⁷pfu/ml) diluted to 1×10⁷pfu/ml in DMEM w/10% FBS and mixed by pipetting. 5µl was applied to each film sample by pipet, and spread to ensure maximum contact, using a 10% organic soil load. After 2 hrs. contact time, virus was recovered by washing with DMEM (Dulbecco's Modified Eagle Medium) containing 10% serum. Resulting dilutions were used to infect 5x105 VeroE6 cells, in triplicate. Wells were covered with Noble aga. Plates were incubated for three days at 37°C and 5% CO2. Agar was removed and cells were stained with Crystal Violet. Plaques were counted in wells containing the lowest countable dilution and recovered virus concentrations were calculated. Results show that a > 99.999% reduction in virus concentration (> 5 log₁₀ CFU reduction).

### (Example 2)

The example was conducted to evaluate the effectiveness in inhibiting Aspergillus niger black mold according to the AATCC Test Method 30-2004 Test III. Aspergillus niger, ATCC#6275, was harvested into sterile distilled water containing glass beads. The flask was shaken to bring the spores into suspension for use as the test inoculum. One (1.0) mL of the inoculum was evenly distributed over the surface of Mineral Salts Agar plates. Film samples of SSBC-1 and SSBC-2 (constructed similar to Example 1) were placed onto the inoculated agar surface. After placement, 0.2 mL of the inoculum was distributed over the surface of each disc. A viability plate of the spore suspension was prepared on Mineral Salts Agar with 3% glucose. A positive growth control was prepared using an untreated cotton duck fabric on Mineral Salts Agar and set up in the same manner as the test items. All samples were incubated at 28°C ± 1°C for 14 days.

The viability plate had acceptable fungal growth as expected confirming the viability of the inoculum. The sample with 26% sulfonation showed microscopic growth on 10% of the sample surface. The other 5 test samples showed no growth or microscopic growth on 1% of the surface. A control sample (without the antimicrobial composition) showed macroscopic growth on 100% of the surface.

### (Example 3)

Various solutions of SSBCs were prepared by dissolving dried sheets of the SSBCs in Table 1 in various solvent systems as shown in Table 2. Prepared solutions have solid contents ranging from 1 - 20%, and 5 - 8% of the solid content for spraying. Table 3 shows the solubility parameters of the various solvent mixtures employed.

**Table 2**

| Solvent Mixtures | Solvent-I | Solvent-II | Ratio of solvent-I:solvent-II |
|---|---|---|---|
| SolMix-1a | 1-Butanol | Heptane | 03:01 |
| SolMix-1b | 2-Propanol | Ethyl ether | 02:01 |
| SolMix-1c | 2-Propanol | Heptane | 02:01 |
| SolMix-1d | 2-Propanol | tert-Butvl methyl ether | 02:01 |
| SolMix-1e | Ethyl acetate | Heptane | 03:01 |
| SolMix-1f | Acetone | tert-Butyl methyl ether | 03:01 |
| SolMix-1h | Acetone | Heptane | 03:01 |
| SolMix-1i | Butyl acetate | Ethyl ether | 03:01 |
| SolMix-1i | 1-Propanol | Heptane | 03:01 |
| SolMix-1k | 2-Propanol | Heptane | 03:01 |

**Table 3**

| Solvent Mixtures | Acetone (Wt.%) | 2-Propanol (Wt.%) | Heptane (Wt.%) | δ_{D} | δ_{P} | δ_{H} |
|---|---|---|---|---|---|---|
| SolMix-2a | 39.04 | 24.58 | 36.39 | 15.50 | 5.56 | 6.76 |
| SolMix-2b | 19.84 | 29.96 | 50.20 | 15.49 | 3.89 | 6.30 |
| SolMix-2c | 37.62 | 25.00 | 37.38 | 15.50 | 5.44 | 6.73 |
| SolMix-2d | 20.24 | 29.96 | 49.80 | 15.49 | 3.93 | 6.33 |
| SolMix-2e | 19.96 | 19.76 | 60.28 | 15.44 | 3.28 | 4.64 |
| SolMix-2f | 20.24 | 39.68 | 40.08 | 15.54 | 4.53 | 7.92 |
| SolMix-2h | 10 | 10 | 80 | 15.37 | 1.65 | 2.34 |
| SolMix-2i | 15 | 15 | 70 | 15.41 | 2.48 | 3.51 |
| SolMix-2j | - | 40 | 60 | 15.50 | 2.44 | 6.56 |
| SolMix-2k | 40 | - | 60 | 15.38 | 4.16 | 2.80 |

The sulfonated block copolymer (either SSBC1 or SSBC2) was either fully dissolved forming a homogeneous clear solution in the solvent mixture (SolMix-2a and SolMix2b), partially dissolved forming an almost clear solution (SolMix-2f) or somewhat cloudy solution samples. The solubility was dependent on the solvent mixture, and not the selection of the SSBC sample. However, all samples were in a form that can be used for mixing with other components forming an antimicrobial composition that can be sprayed onto surfaces forming an antimicrobial wipe, or for the dipping of a fibrous substrate forming a pre-saturated wet, or a pre-saturated dry wipe after evaporation of the solvent system.

### (Example 4)

Polyether-ether-ketone is sulfonated by sulfuric acid and then purified to obtain a sulfonated polyether-ether-ketone. An antimicrobial wipe is prepared with the sulfonated polyether-ether-ketone and the solvent system. The antimicrobial composition can be sprayed onto a fabric of pre-determined size forming an antimicrobial wipe.

As used herein, the term "comprising" means including elements or steps that are identified following that term, but any such elements or steps are not exhaustive, and an embodiment can include other elements or steps. Although the terms "comprising" and "including" have been used herein to describe various aspects, the terms "consisting essentially of" and "consisting of" can be used in place of "comprising" and "including" to provide for more specific aspects of the disclosure and are also disclosed.

## Claims

1. An antimicrobial wipe for protecting a surface, comprising:
a wet wipe having a substrate impregnated with a sufficient amount of an antimicrobial composition which comprises:
(i) 0.1 to 50 wt.% of a sulfonated block copolymer having an ion exchange capacity (IEC) of at least 0.5 meq/g, and
(ii) 50 to 99.90 wt.% of a solvent system containing a mixture of two or more solvents selected from the group consisting of organic solvents, inorganic solvents, terpene-based compounds, and decarboxylated rosin acids, wherein each solvent has at least one of:
a) at least one OH group,
b) at least one ester and / or ether group, and
c) 10 or more carbon atoms;
wherein the antimicrobial wipe after wiping with a shear force onto the surface to be protected, forms a coating layer; and
wherein the coating layer causes reduction in a microbe concentration by at least 1 logic CFU (colony forming units) within 120 minutes upon contact with the coating layer.

2. An antimicrobial wipe for protecting a surface, comprising:
a dry wipe having a substrate impregnated with a sufficient amount of a sulfonated block copolymer having an ion exchange capacity (IEC) of at least 0.5 meq/g;
wherein the substrate containing the sulfonated block copolymer after being in contact with a solvent system pre-applied onto the surface to be protected or the substrate itself, is dissolved into a solvent system for the sulfonated block copolymer to be imparted from the substrate onto the surface upon contact, forming a coating layer;
wherein the solvent system contains a mixture of two or more solvents selected from the group consisting of organic solvents, inorganic solvents, terpene-based compounds, and decarboxylated rosin acids, wherein each solvent has at least one of:
a) at least one OH group,
b) at least one ester and / or ether group, and
c) 10 or more carbon atoms; and
wherein the coating layer causes reduction in a microbe concentration by at least 1 log₁₀ CFU (colony forming units) within 120 minutes upon contact with the coating layer.

3. The antimicrobial wipe of any of claims 1-2, wherein the solvent system has a total solubility parameter (*δ_{T}*) of greater than or equal to 14.5.

4. The antimicrobial wipe of any of claims 1-2, wherein the solvent system contains a mixture of two different solvents in a weight ratio of 10:90 to 90:10, wherein the mixture is selected from: ethyl acetate and 1-propanol, tert-butyl methyl ether and 1-propanol, 2-ethyl butyl acetate and 1-propanol, butyl lactate and butanol, 1-decanol and butanol, 1-undecanol and isopropyl myristate, 1-propanol and isopropyl myristate, decanal and isopropyl myristate, 1-undecanol and butanol, 2-ethyl butyl acetate and 1-propanol, ethyl lactate and butanol, isododecane and butanol, decanal and butanol, 1-undecanol and isododecane, decanal and isododecane, n-dodecane and butanol, ethyl glycol acetate and 1-propanol, ethyl glycol acetate and butanol, 1-pentanol and isopropyl myristate, 1-undecanol and 1-pentanol, 2-ethyl hexanoic acid and ethyl acetate, ethylene glycol mono hexyl ether and 1-propanol, ethylene glycol mono hexyl ether and butanol, ethylene glycol mono hexyl ether and ethyl lactate, ethylene glycol mono hexyl ether and ethyl glycol acetate, ethylene glycol mono hexyl ether and 1-pentanol, ethylene glycol mono hexyl ether and 1-decanol, ethylene glycol mono hexyl ether and isododecane, ethylene glycol mono hexyl ether and decanal, isododecane and 1-pentanol, 2-ethyl hexanoic acid and ethylene glycol mono hexyl ether, 2-ethyl hexanoic acid and ethyl acetate, ethylene glycol mono hexyl ether and ethanol, isododecane and ethanol, ethyl glycol acetate and ethanol, and neopentyl glycol and ethanol.

5. The antimicrobial wipe of any of claims 1 - 2, wherein the solvent system has a Hildebrand Solubility Parameter (HSP) a δD of 15 to 16; a δP of 1 to 7; and a δH of 2 to 9.

6. The antimicrobial wipe of any of claims 1-2, wherein the coating layer has a thickness of greater than or equal to 1 µm.

7. The antimicrobial wipe of any of claims 1-2, wherein the coating layer causes reduction in a microbe concentration by at least 3 log₁₀ CFU within 30 minutes upon contact with the coating layer.

8. The antimicrobial wipe of any of claims 1-2, wherein the sulfonated block copolymer has at least one of: a degree of sulfonation of 10 to 100 mol%, and a molecular weight (Mₚ) of 25 to 500 kg/mol.

9. The antimicrobial wipe of any of claims 1 - 2, wherein the sulfonated block copolymer has an ion exchange capacity of (IEC) of 0.5 to 2.6 meq/g.

10. The antimicrobial wipe of any of claims 1 - 2, wherein the sulfonated block copolymer is a sulfonated styrenic block copolymer obtained by sulfonation of a styrenic block copolymer precursor having a general configuration of: A-B-A, (A-B)ₙ(A), (A-B-A)ₙ, (A-B-A)ₙX, (A-B)ₙX, A-D-B, A-B-D, A-D-B-D-A, A-B-D-B-A, (A-D-B)ₙA, (A-B-D)ₙA (A-D-B)ₙX, (A-B-D)ₙX, (A-D-B-D-A)ₙX, (A-B-D-B-A)ₙX or mixtures thereof, where n is an integer from 2 to 30, and X is a residue of a coupling agent; and wherein:
each block A is derived from polymerized para-substituted styrene monomers selected from the group consisting of para-methylstyrene, para-ethylstyrene, para-n-propylstyrene, para-iso-propylstyrene, para-n-butylstyrene, para-sec-butylstyrene, para-iso-butylstyrene, para-t-butylstyrene, isomers of para-decylstyrene, isomers of para-dodecylstyrene, and mixtures thereof;
each block B is derived from the polymerized vinyl aromatic monomers selected from the group consisting of unsubstituted styrene, ortho-substituted styrene, meta-substituted styrene, alpha-methylstyrene, 1,1-diphenylethylene, 1,2-diphenylethylene, and mixtures thereof; and
each block D is derived from the polymerized conjugated diene monomers selected from the group consisting of isoprene, 1,3-butadiene, 2,3-dimethyl-1,3-butadiene, 1-phenyl-1,3-butadiene, 1,3-pentadiene, 1,3-hexadiene, 3-butyl-1,3-octadiene, farnesene, myrcene, piperylene, cyclohexadiene, and mixtures thereof.

11. The antimicrobial wipe of any of claims 1-2, wherein the substrate is selected from the group consisting of towelette, absorbent sheet, fabric, sponge, paper, absorbent polymer, and mixtures thereof.

12. A method for protecting a surface, comprising:
providing an wet wipe having a substrate impregnated with a sufficient amount of an antimicrobial composition comprising a sulfonated block copolymer having an ion exchange capacity (IEC) of at least 0.5 meq/g, and a solvent system, wherein the sulfonated block copolymer is dissolved in the solvent system containing a mixture of two or more solvents selected from the group consisting of organic solvents, inorganic solvents, terpene-based compounds, and decarboxylated rosin acids, and wherein each solvent has at least one of: a) at least one OH group, b) at least one ester and / or ether group, and c) 10 or more carbon atoms; and
wiping the surface with the wet wipe by shear force for the antimicrobial composition to be imparted from the wet wipe onto the surface, forming a coating layer;
wherein the coating layer causes reduction in a microbe concentration by at least 1 log10 CFU (colony forming units) within 120 minutes upon contact with the coating layer.

13. A method for protecting a surface, comprising:
providing a dry wipe having a substrate impregnated with a sufficient amount of a sulfonated block copolymer having an ion exchange capacity (IEC) of at least 0.5 meq/g;
pre-applying a solvent system onto the surface to be protected, the solvent system contains a mixture of two or more solvents selected from the group consisting of organic solvents, inorganic solvents, terpene-based compounds, and decarboxylated rosin acids, wherein each solvent has at least one of: a) at least one OH group, b) at least one ester and / or ether group, and c) 10 or more carbon atoms;
contacting the substrate with the surface having the pre-applied solvent system, for the sulfonated block copolymer to be imparted from the substrate to the surface by being dissolved in the pre-applied solvent system, forming a coating layer on the surface; and
wherein the coating layer causes reduction in a microbe concentration by at least 1 log10 CFU (colony forming units) within 120 minutes upon contact with the coating layer.

14. A method for protecting a surface, comprising:
providing a dry wipe having a substrate impregnated with a sufficient amount of a sulfonated block copolymer having an ion exchange capacity (IEC) of at least 0.5 meq/g;
pre-applying a solvent system onto the substrate to dissolve the sulfonated block copolymer, the solvent system contains a mixture of two or more solvents selected from the group consisting of organic solvents, inorganic solvents, terpene-based compounds, and decarboxylated rosin acids, wherein each solvent has at least one of: a) at least one OH group, b) at least one ester and / or ether group, and c) 10 or more carbon atoms;
contacting the substrate with the surface to be protected, for the sulfonated block copolymer dissolved in the pre-applied solvent system to be imparted from the substrate to the surface, forming a coating layer on the surface; and
wherein the coating layer causes reduction in a microbe concentration by at least 1 log10 CFU (colony forming units) within 120 minutes upon contact with the coating layer.

15. The method of any of claims 12 - 14, wherein the solvent system has an ion exchange capacity of (IEC) of 0.5 to 2.6 meq/g.
